# EUROPÄISCHE PATENTSCHRIFT

(11) **EP 3 365 044 B1**
(45) Veröffentlichungstag und Bekanntmachung des Hinweises auf die Patenterteilung: **01.05.2024**
(21) Anmeldenummer: 16784797.9
(22) Anmeldetag: 21.10.2016
(51) Int. Cl.: A61M 1/36

(54) **VORRICHTUNG, SYSTEM UND VERFAHREN ZUR DRUCKBASIERTEN ERKENNUNG EINES GERINNSELS**
DEVICE, SYSTEM, AND METHOD FOR THE PRESSURE-BASED DETECTION OF A CLOT
DISPOSITIF, SYSTÈME ET PROCÉDÉ POUR LA DÉTECTION BASÉE SUR LA PRESSION D'UN CAILLOT

(30) Priorität: 21.10.2015 DE 102015013610
(43) Veröffentlichungstag der Anmeldung: 29.08.2018
(73) Patentinhaber: Fresenius Medical Care Deutschland GmbH, 61352 Bad Homburg (DE)
(72) Erfinder: THYS, Martin, 97508 Grettstadt (DE); NOACK, Joachim, 97616 Bad Neustadt (DE)
(74) Vertreter: Herrmann, Uwe
(86) Internationale Anmeldenummer: PCT/EP2016/001762
(87) Internationale Veröffentlichungsnummer: WO 2017/067668

(56) Entgegenhaltungen:
- DE-A1-102006 032 815
- DE-A1-102009 018 664
- DE-A1-102009 024 606
- DE-B3- 10 355 042

## Beschreibung

Die vorliegende Erfindung betrifft eine Vorrichtung zur druckbasierten Erkennung eines Gerinnsels, sowie ein System mit wenigstens einer solchen Vorrichtung, wobei das System wenigstens ein Blutbehandlungsgerät umfasst, das über wenigstens einen extrakorporalen Kreislauf verfügt, mit dem zumindest eine zyklisch arbeitende Pumpe zur Förderung der in dem extrakorporalen Kreislauf befindlichen Flüssigkeit zusammenwirkt.

Aus dem Stand der Technik ist es bekannt, dass Blutbehandlungsvorrichtungen, wie beispielsweise Hämofiltrationsgeräte, Hämodiafiltrationsgeräte, Hämodialysegeräte in dem extrakorporalen Kreislauf einen Gerinnselfänger aufweisen, der die Aufgabe hat, vor der Infusion des Blutes aus dem extrakorporalen Kreislauf in den Patienten dieses mittels Filtration von gefährlich großen Gerinnseln zu befreien.

Die Verwendung von Gerinnselfängern bringt zwar den Vorteil mit sich, dass das Eintreten derartiger Gerinnsel in den Blutkreislauf des Patienten verhindert wird, ist jedoch mit dem Nachteil behaftet, dass der Gerinnselfänger selbst im fließenden Blut auf dieses negative Auswirkungen hat. Trifft das fließende Blut auf den Gerinnselfänger, entstehen Turbulenzen. Bedingt durch diese Turbulenzen laufen im Blut physikalische sowie auch biochemische Prozesse an, die dazu führen können, dass die Koagulation des Blutes aktiviert wird bzw. Blutzellen zerstört werden. Bei einer Koagulation des Blutes können stromabwärts des Gerinnselfängers Koagel entstehen, die eine Gefährdung für den Patienten darstellen können, indem diese Blutgefäße verstopfen oder deren Durchströmung behindern.

Kommt es zu einer Zerstörung von Blutzellen (Hämolyse), kann das Blut seine Funktion in vivo nicht mehr ausreichend ausführen, was für den Patienten ebenfalls eine gefährdende Situation darstellen kann.

Da die Vorteile des Gerinnselfängers dessen Nachteile überwiegen, werden diese üblicherweise eingesetzt.

Im Rahmen einer üblichen Blutbehandlung wird das Blut über die arterielle Leitung des extrakorporalen Kreislaufs in den extrakorporalen Kreislauf gesaugt, darin z.B. in einem Dialysator etc. behandelt und über die venöse Leitung in die Vene des Patienten gepumpt. In diesem Falle ist es bekannt, einen Gerinnselfänger in der venösen Leitung vorzusehen, d.h. in dem Leitungsabschnitt des extrakorporalen Kreislaufes, durch den das Blut dem Patienten wieder zugeführt wird.

Um das in dem extrakorporalen Kreislauf befindliche Blut nach der Blutbehandlung wieder dem Patienten zu zuführen, erfolgt eine Reinfusion dieses Blutes aus dem extrakorporalen Kreislauf zum Patienten. Dabei ist es bekannt, dass das nach der Blutbehandlung noch im extrakorporalen Kreislauf befindliche Blut mittels Verdrängung durch eine Austauschflüssigkeit sowohl durch die arterielle als auch durch die venöse Leitung des extrakorporalen Kreislaufes in den Patienten gefördert wird.

Während dieser auf die Behandlung folgenden und relativ zur Blutbehandlung zeitlich deutlich kürzer andauernden Reinfusion des Blutes findet somit in der arteriellen Leitung hinsichtlich der Blutflussrichtung eine Flussumkehr statt. Würde auch in der arteriellen Leitung ein Gerinnselfänger verbaut, hätte dies zwar im Rahmen der Reinfusion den Vorteil, dass gefährlich große Gerinnsel von dem Wiedereintritt in den Patientenblutkreislauf gehindert werden. Allerdings hätte der Gerinnselfänger während der vergleichsweise langen Behandlungszeit die oben beschriebenen Nachteile im Hinblick auf die Entstehung von Turbulenzen im Blut und damit im Hinblick auf eine etwaige Gerinnselbildung stromabwärts des Gerinnselfängers oder eine etwaige Hämolyse. Vor diesem Hintergrund wird davon abgesehen, in der arteriellen Leitung des extrakorporalen Kreislaufes einen Gerinnselfänger einzusetzen.

Die WO 2013/000777A1 offenbart die Messung von Drucksignalen in einem extrakorporalen Kreislauf. Um ein gemessenes überlagertes Drucksignal zu ermitteln, werden aus diesem Drucksignal von der Blutpumpe erzeugte Drucksignale heraus gefiltert. Aus der DE 24 41 210 A1 ist ein Verfahren bekannt, bei dem zyklisch beim Abziehen des Blutes von dem Patienten in den extrakorporalen Kreislauf die venöse Leitung und beim Zuleiten des Blutes zu dem Patienten aus dem extrakorporalen Kreislauf die arterielle Leitung abgesperrt wird und aus der Dauer des Zyklus auf etwaige Unregelmäßigen geschlossen wird. DE 103 55 042 B3 offenbart Verfahren zum Erkennen von Störungen des Blutflusses in einem extrakorporalen Blutkreislauf auf Grund der Veränderung des Phasenwinkels von Oberschwingungen eines oszillierenden Drucksignals.

Der vorliegenden Erfindung liegt die Aufgabe zugrunde, eine Vorrichtung und ein System bereitzustellen, bei dem der Eintritt von Gerinnseln im Rahmen der Reinfusion aus dem extrakorporalen Kreislauf in den Patienten erkannt werden kann.

Diese Aufgabe wird durch eine Vorrichtung mit den Merkmalen des Anspruchs 2. 1 und durch ein System mit den Merkmalen des Anspruchs 9 gelöst.

Danach ist vorgesehen, dass die Vorrichtung wenigstens eine zyklisch arbeitende Pumpe zur Förderung der in dem extrakorporalen Kreislauf befindlichen Flüssigkeit sowie wenigstens einen Druckaufnehmer umfasst, der angeordnet ist, um in einem aktuellen Förderzyklus der Pumpe den aktuellen Druckverlauf über die Zeit in der von der Pumpe geförderten Flüssigkeit zu ermitteln, wobei die Vorrichtung wenigstens einen Speicher aufweist, in dem wenigstens ein Referenzdruckverlauf abgespeichert ist, der auf wenigstens einem Druckverlauf über die Zeit basiert, der in einem dem aktuellen Förderzyklus der Pumpe vorausgehenden Förderzyklus der Pumpe ermittelt wurde, und wobei die Vorrichtung wenigstens eine Auswerteeinheit umfasst, die derart ausgebildet ist, dass diese den aktuellen Druckverlauf mit dem Referenzdruckverlauf zeitentsprechend vergleicht.

Unter einer "zyklisch arbeitenden Pumpe" ist eine Pumpe zu verstehen, deren Förderleistung und/oder deren durch die Förderleistung bedingter Druckverlauf in der geförderten Flüssigkeit nicht zeitlich konstant ist, sondern vorzugsweise in zeitlich aufeinander folgenden Arbeitszyklen verläuft. Vorzugsweise wird darunter eine Pumpe verstanden, die in aufeinander folgenden Perioden bzw. Zyklen pumpt.

Die Vorrichtung weist des Weiteren wenigstens einen Speicher auf, in dem wenigstens ein Referenzdruckverlauf abgespeichert ist, der auf einem früheren Druckverlauf über die Zeit basiert. Dieser frühere Druckverlauf wurde mittels des Druckaufnehmers in der von der Pumpe geförderten Flüssigkeit in wenigstens einem dem aktuellen Druckverlauf vorausgehenden Förderzyklus der Pumpe ermittelt.

Vorzugsweise ist vorgesehen, dass durch die Auswerteeinheit zumindest eine Maßnahme veranlasst wird, wenn der aktuelle Druckverlauf den Referenzdruckverlauf erreicht oder diesen übersteigt.

Unter einem "zeitentsprechenden Vergleich" ist zu verstehen, dass entsprechende Zeitpunkte des Referenzdruckverlaufs mit entsprechenden Zeitpunkten des aktuellen Druckverlaufes verglichen werden. So wird beispielsweise der Druck zum Zeitpunkt t = 10 Sekunden des Referenzdruckverlaufs mit dem gleichen Zeitpunkt (t = 10 Sekunden) des aktuellen Druckverlaufes verglichen. Wird dabei festgestellt, dass der Wert des aktuellen Druckverlaufes den Wert des Referenzdruckverlaufes erreicht oder gar übersteigt, ist vorzugsweise vorgesehen, dass die Auswerteeinheit wenigstens eine Maßnahme veranlasst, wie beispielsweise ein Stoppen der Blutreinfusion, z.B. durch das Schließen eines Ventils des extrakorporalen Kreislaufes, das Anhalten der genannten Pumpe oder dergleichen.

Ein Gerinnsel von Gefahr bringender Größe verringert den effektiven Flussquerschnitt und kann bei der Förderung zum Patienten aus dem extrakorporalen Kreislauf hinaus den Patientenkonnektor bzw. die daran angeschlossene Nadel verstopfen. Der sich ergebende Druckanstieg wird erfasst und als bevorzugte Maßnahme wird die Förderung sofort eingestellt, um zu verhindern, dass das Gerinnsel in den Patientenblutkreislauf gelangt. Da ein Gerinnsel mechanisch flexibel ist, kann ein Gerinnsel, das nur wenig größer ist als die davor befindliche Engstelle unter Umständen in Bruchteilen einer Sekunde durch den Patientenkonnektor bzw. die daran angeschlossene Nadel und ggf. weiter in die Fistel gepresst werden. Die erfindungsgemäße Drucküberwachung ist ausreichend sensitiv, um eine durch ein Gerinnsel bedingte Druckveränderung zu erfassen und dann entsprechende Maßnahmen zu ergreifen.

Da das Förderverhalten einer zyklisch arbeitenden Pumpe, wie beispielsweise einer Schlauchrollenpumpe zeitlich nicht gleichmäßig ist, scheidet die Überwachung wegen zeitweise zu geringer Sensitivität auf eine feste Druckgrenze hin aus.

Der durch die zyklisch arbeitende Pumpe verursachte über den zeitlichen Verlauf ungleichmäßige Volumenstrom des zu fördernden Mediums (Blut, Austauschflüssigkeit oder ein Gemisch aus beiden) wiederholt sich zyklisch je Aktivierung eines an der Förderung beteiligten Elementes, wie beispielsweise einer Schlauchrolle, die den Schlauch des extrakorporalen Kreislaufes über eine Teilstrecke ihrer Bewegung zusammenpresst und so die Förderung der Flüssigkeit bewirkt.

Die erfindungsgemäße Vorrichtung bzw. das System basiert darauf, den aktuellen Druckverlauf in einem aktuell laufenden Zyklus jeweils mit einem Referenzdruckverlauf zu vergleichen, um einen durch ein Gerinnsel induzierten Druckanstieg sehr rasch erkennen zu können.

Vorzugsweise wird ein Teilbereich eines Gesamtdruckverlaufes, der bei periodisch arbeitenden Pumpen eine Periode oder eine Teilperiode umfasst, mit einem Referenzdruckverlauf verglichen.

Bei der Pumpe kann es beispielsweise um eine peristaltische Pumpe bzw. um eine Schlauchrollenpumpe handeln. Von der Erfindung sind jedoch auch andere Pumpen umfasst, die zyklisch arbeiten. Von der Erfindung sind alle Pumpen umfasst, die im zeitlichen Verlauf pulsieren bzw. einen zyklischen Druckverlauf besitzen bzw. eine periodisch schwankende Förderrate aufweisen, d.h. nicht gleichmäßig fördernde Pumpen. Ein weiteres Beispiel für eine solche Pumpe ist eine Membranpumpe.

Vorzugsweise basiert der Referenzdruckverlauf auf einem gegenüber dem aktuellen Druckverlauf phasenverschobenen Druckverlauf. So ist es beispielsweise denkbar, den Druckverlauf während einer halben Umdrehung der Pumpe zu messen und diesen ggf. mit einem Zuschlag für die darauffolgende halbe Umdrehung als Referenzdruckverlauf heranzuziehen. Liegt der Druck in dem aktuellen Förderzyklus oberhalb des Referenzwertverlaufs bzw. Schwellenwertverlaufs oder erreicht diesen, werden vorzugsweise eine oder mehrere Maßnahmen durchgeführt, wie beispielsweise die Pumpe gestoppt, ein Ventil geschlossen und/oder ein Alarm ausgelöst.

Vorzugsweise wird der Referenzdruckverlauf basierend auf dem dem aktuellen Druckverlauf unmittelbar vorausgegangenen Druckverlauf bestimmt. Somit ist nur ein vergleichsweise geringer Datenspeicheraufwand notwendig.

Grundsätzlich kann es sich bei dem Referenzdruckverlauf um einen Druckverlauf aus der Vergangenheit, d.h. um einen Druckverlauf handeln, der zu einem beliebigen Zeitpunkt dem aktuellen Druckverlauf vorausgegangen ist. Vorzugsweise handelt es sich bei dem Referenzdruckverlauf um einen dem aktuellen Druckverlauf unmittelbar vorausgegangenen Druckverlauf, wie z.B. um den Druckverlauf in einer unmittelbar vorausgegangenen Periode oder Teilperiode der Pumpenförderung oder um einen oder mehrere von Druckverläufen oder Teilen davon, die in davor, d.h. weiter zurück liegenden Perioden oder Teilperioden der Pumpenförderung ermittelt wurden.

Geht man von einer Pumpe mit zwei Schlauchrollen aus, die dementsprechend pro Umdrehung zwei weitestgehend identische Förderzyklen hat, würde dies bedeuten, dass der aktuelle Druckverlauf immer nur mit dem vorausgehenden Druckverlauf verglichen werden muss, der während der vorhergehenden halben Umdrehung der Pumpe ermittelt wurde.

Entsprechendes gilt selbstverständlich auch für sämtliche andere nicht stetig fördernde Pumpen, wie etwa zyklisch arbeitenden Pumpen und nicht nur für Pumpen, die pro vollständiger Umdrehung zwei Förderzyklen aufweisen, wie etwa Membranpumpen.

In einer weiteren Ausgestaltung der Erfindung ist vorgesehen, dass der Referenzdruckverlauf durch einen in einem vorausgegangenen Förderzyklus der Pumpe ermittelten Druckverlauf zuzüglich eine Zuschlages auf diesen Druckverlauf gebildet wird.

Denkbar ist, auf diesen gemessenen Druckverlauf einen absoluten Zuschlag oder einen relativen Zuschlag zuzugeben. Denkbar ist es, dass der Referenzdruckverlauf dadurch gebildet wird, dass der Druckverlauf um 5 bis 50% angehoben wird. Dieser Zuschlag bringt den Vorteil mit sich, dass bei kleinen Unterschieden zwischen fördernden Elementen der Pumpe keine Fehlalarme erzeugt werden.

In weiterer Ausgestaltung der Erfindung ist vorgesehen, dass der extrakorporale Kreislauf zumindest eine arterielle Leitung und zumindest eine venöse Leitung aufweist und dass der Druckaufnehmer in der arteriellen und/oder in der venösen Leitung angeordnet ist.

Vorzugsweise befindet sich der Druckaufnehmer in der arteriellen Leitung. In der venösen Leitung ist vorzugsweise kein Druckaufnehmer angeordnet. Dafür ist dort in einer bevorzugten Ausgestaltung ein Gerinnselfänger angeordnet, der seinerseits in der arteriellen Leitung fehlt.

Die Erfindung kann auch so ausgeführt sein, dass überhaupt kein Gerinnselfänger angeordnet ist. Grundsätzlich ist es auch denkbar und von der Erfindung umfasst, sowohl in der arteriellen Leitung als auch in der venösen Leitung oder nur in einer dieser Leitungen einen Gerinnselfänger anzuordnen.

Grundsätzlich ist die Erfindung in der arteriellen Leitung, in der venösen Leitung oder sowohl in der arteriellen als auch in der venösen Leitung anwendbar, d.h. umfasst sämtliche dieser Ausgestaltungen.

In einer weiteren Ausgestaltung der Erfindung ist die Auswerteeinheit so ausgebildet, dass diese als Maßnahme z. B. ein Ventil im extrakorporalen Kreislauf schließt und/oder die Pumpe stoppt, so dass das Gerinnsel nicht weiterbefördert wird und nicht in den Blutkreislauf des Patienten gelangt.

Alternativ oder zusätzlich kann ein Hinweis an einen Nutzer ausgegeben werden, wie bspw. ein Alarmsignal, der/das auf das Vorhandensein eines Gerinnsels hinweist.

Die vorliegende Erfindung betrifft des Weiteren ein System mit den Merkmalen des Anspruchs 10. Das System umfasst wenigstens eine Vorrichtung gemäß der vorliegenden Erfindung und weist wenigstens einen extrakorporalen Kreislauf auf, der vorzugsweise über wenigstens einen Konnektor verfügt, mittels dessen der extrakorporale Kreislauf mit dem Patienten verbindbar ist. Mit dem extrakorporalen Kreislauf wirkt die wenigstens eine zyklisch arbeitende Pumpe der Vorrichtung zur Förderung der in dem extrakorporalen Kreislauf befindlichen Flüssigkeit zusammen.

Das System kann durch ein Blutbehandlungsgerät gebildet werden oder dieses umfassen. Im ersten Fall sind die Komponenten des Systems Bestandteile des Blutbehandlungsgerätes, im zweiten Fall ist wenigstens eine Komponente des Systems kein Bestandteil des Blutbehandlungsgerätes, sondern wird durch eine externe Einheit gebildet.

Bei dem Blutbehandlungsgerät kann es sich bspw. um ein Hämofiltrations-, Hämodialyse-, Hämodiafiltrations-, Apheresegerät oder um eine Vorrichtung zur Medikation des Patienten handeln.

Die vorliegende Offenbarung betrifft des Weiteren ein Schlauchset, das zur Verwendung in einer Vorrichtung gemäß einem der Ansprüche 1 bis 9 oder zur Verwendung in einem System gemäß einem der Ansprüche 10 bis 12 geeignet und bestimmt ist. Das Schlauchset bildet den extrakorporalen Kreislauf oder einen Abschnitt von diesem, wobei das Schlauchset mit wenigstens einem Konnektor zur Verbindung des extrakorporalen Kreislaufes mit einem Patienten versehen ist und wobei das Schlauchset eine arterielle Leitung und eine venöse Leitung aufweist und wobei kein Gerinnselfänger in der arteriellen Leitung oder kein Gerinnselfänger in der venösen Leitung oder kein Gerinnselfänger in beiden Leitungen angeordnet ist. Das Schlauchset kann somit ohne Gerinnselfänger ausgebildet sein.

Es ist vorgesehen, dass in einem aktuellen Förderzyklus der Pumpe der aktuelle Druckverlauf über die Zeit in der von der Pumpe geförderten Flüssigkeit ermittelt wird. Dieser aktuelle Druckverlauf wird mit dem genannten Referenzdruckverlauf verglichen. Wie oben ausgeführt, wird wenigstens eine Maßnahme veranlasst, wenn der aktuelle Druckverlauf den Referenzdruckverlauf übersteigt oder diesen erreicht. Dabei ist vorzugsweise vorgesehen, dass zur Durchführung dieses Verfahrens die Pumpe, die sich vorzugsweise in dem arteriellen Teil des extrakorporalen Kreislaufes befindet in entgegengesetzter Richtung zur üblichen Förderrichtung während der Blutbehandlung läuft. Somit wird die Flüssigkeit in der arteriellen Leitung nicht von dem Konnektor weg, sondern zu dem Konnektor hin befördert.

Wie oben ausgeführt, erfolgt die Druckmessung in der arteriellen und/oder venösen Leitung. Bevorzugt ist es, wenn die Druckmessung in der Leitung erfolgt, in der kein Gerinnselfänger angeordnet ist.

Bei der genannten Maßnahme kann es sich um das Absperren des extrakorporalen Kreislaufes durch ein oder mehrere Ventile, um das Anhalten der Pumpe, um die Ausgabe eines Hinweises an den Nutze wie bspw. eines Alarmes oder um eine Mehrzahl dieser Maßnahmen handeln.

Vorzugsweise ist vorgesehen, dass die Flüssigkeit, d.h. das Blut, das Gemisch aus Flüssigkeit und Austauschflüssigkeit bzw. die Austauschflüssigkeit selbst, die aus dem extrakorporalen Kreislauf gefördert wird, auf der arteriellen Seite nicht durch einen Gerinnselfänger geleitet wird.

Der Referenzdruckverlauf kann auf einem gegenüber dem aktuellen Druckverlauf phasenverschobenem Drucklauf basieren. Er kann durch einen in dem vorausgegangenen Förderzyklus der Pumpe ermittelten Druckverlauf zzgl. eines Zuschlages auf diesen Druckverlauf gebildet werden.

In einer bevorzugten Ausgestaltung der Erfindung betrifft die vorliegende Erfindung somit ein System zur Reinfusion von Blut aus einem extrakorporalen Blutkreislauf zurück in den Patienten, ohne dass ein Gerinnselfänger eingesetzt werden muss. Durch die Drucküberwachung wird sichergestellt, dass bei Auftreten eines erhöhten Druckwertes durch ein größeres Gerinnsel Gegenmaßnahmen eingeleitet werden, wie bspw. ein Pumpenstopp oder das Schließen eines Ventils, worunter auch das Schließen einer Schlauchklemme zu verstehen ist.

In einer bevorzugten Ausgestaltung der Erfindung wird zur Drucküberwachung gemäß der Erfindung, der aufgrund der Schlauchpumpe im extrakorporalen Blutkreislauf modulierte Druckverlauf, der um eine halbe Umdrehung phasenverschoben ist, als Referenzdruckverlauf bzw. als Grenzwertkurve herangezogen.

Weitere Einzelheiten und Vorteil der Erfindung werden anhand eines in der Zeichnung beschriebenen Ausführungsbeispiels näher erläutert.

Es zeigen:
- Fig. 1:: Eine stark vereinfachte schematische Ansicht eines Blutbehandlungsgerätes mit extrakorporalem Blutkreislauf,
- Fig. 2:: eine weitere Ansicht eines Blutbehandlungsgerätes mit extrakorporalem Blutkreislauf und
- Fig. 3:: eine Ansicht des Druckverlaufes über die Zeit für mehrere Förderzyklen.

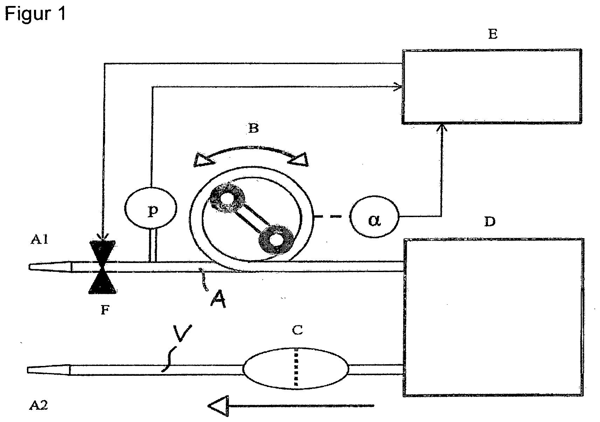

Figur 1 zeigt mit dem Bezugszeichen A1 den arteriellen Patientenanschluss eines extrakorporalen Blutkreislaufes, durch den während der Blutbehandlung Blut mittels der Pumpe B in die arterielle Leitung A gelangt. Durch diesen arteriellen Patientenanschluss, wird das Blut während der Behandlung mittels der Pumpe B in Richtung der Behandlungsvorrichtung D gepumpt, bei der es sich um einen Dialysator handeln kann. Die Behandlungsvorrichtung kann Substitutionsflüssigkeit zur Verfügung stellen. Nach der Behandlung gelangt das Blut gemäß Pfeilrichtung durch den Gerinnselfänger C über die venöse Leitung V zum venösen Patientenanschluss A2.

Das Bezugszeichen F kennzeichnet eine elektrisch betätigte Klemme, mittels derer die arterielle Leitung A absperrbar ist.

Das Bezugszeichen P kennzeichnet einen Drucksensor, der sich zwischen dieser Klemme F und der Blutpumpe B befindet.

Mit dem Bezugszeichen E ist eine Auswerteeinheit bezeichnet, die aus den Ausgangssignalen des Drucksensors sowie eines Drehwinkelsensors, der das Ausgangssignal α liefert, ermittelt, ob eine Druckerhöhung vorliegt, die durch ein Gerinnsel verursacht sein könnte und schließt in diesem Fall sofort die Klemme F.

Während der sich an die Blutbehandlung anschließenden Reinfusion von Blut aus dem extrakorporalen Blutkreislauf wird Blut von der Behandlungsvorrichtung über die Patientenanschlüsse A1 und A2 zurück in den Patienten gefördert.

Dazu wird die Pumpe B in einer zur normalen Richtung entgegengesetzten Richtung (hier: im Uhrzeigersinn) betrieben. Dies führt dazu, dass Blut bzw. die Austauschflüssigkeit nicht wie während der Behandlung vom Anschluss A1 zur Pumpe B, sondern umgekehrt von der Pumpe B zum Anschluss A1 gefördert wird. Das Blut in der venösen Leitung V wird mittels Verdrängung durch eine Austauschflüssigkeit ebenfalls zum Patienten zurückgeführt und zwar über den Anschluss A2. Auf der venösen Seite ergibt sich somit keine Änderung der Flussrichtung im Vergleich zur Flussrichtung während der Blutbehandlung.

Wie dies aus Figur 1 hervorgeht, befindet sich der Gerinnselfänger C nur in der venösen Leitung V. Auf dieser Seite des extrakorporalen Kreislaufes ist somit die Zufuhr eines Gerinnsels zum Patienten durch den Anschluss A2 unwahrscheinlich. Auf der arteriellen Seite befindet sich ein solcher Gerinnselfänger nicht, wie dies aus Figur 1 hervorgeht. Um sicherzustellen, dass auch auf der arteriellen Seite kein Eintritt eines Gerinnsels in den Blutkreislauf des Patienten erfolgt, wird auf der arteriellen Seite das Verfahren durchgeführt.

Die vorliegende Erfindung ist jedoch nicht auf die Anwendung des Verfahrens in der arteriellen Seite beschränkt. Alternativ oder zusätzlich ist die Erfindung auch in der venösen Seite des extrakorporalen Blutkreislaufs anwendbar.

Die Ausführung gemäß Figur 1 beschreibt eine denkbare, jedoch stark vereinfachte Vorrichtung und Verfahrensweise zur Reinfusion.

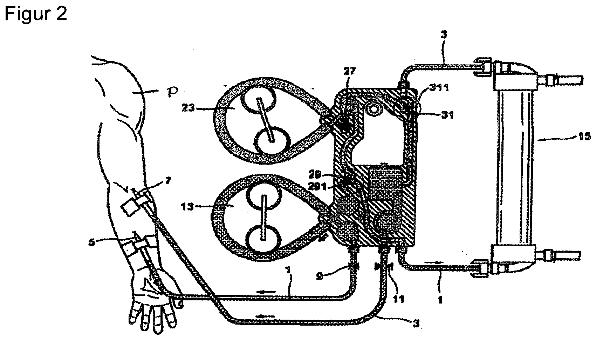

Eine detaillierte Beschreibung einer möglichen Reinfusion findet sich in der DE 10 2009 024 606 A1, auf die hiermit vollumfänglich Bezug genommen wird. Figur 2 zeigt eine Ausführungsform der DE 10 2009 024 606 A1, anhand derer sich ein Beispiel für die Reinfusion wie folgt beschreiben lässt.

Die Bezugszeichen 5, 7 kennzeichnen den arteriellen (5) und den venösen Zugang (7) zum Patienten. Das Bezugszeichen 1 kennzeichnet die arterielle Leitung und das Bezugszeichen 3 die venöse Leitung. Mit dem Bezugszeichen 15 ist ein Dialysator oder eine sonstige Blutbehandlungseinheit gekennzeichnet.

Die arterielle Leitung 1 ist durch die Schlauchklemme 9 und die venöse Leitung 3 durch die Schlauchklemme 11 absperrbar.

Um das Blut aus dem extrakorporalen Blutkreislauf nach der Beendigung der Blutbehandlung in den Patienten zurückzuführen, werden die Klemmen 9, 11 geöffnet und die Blutpumpe 13 sowie die Substiutatpumpe 23 werden betrieben. Die Blutpumpe 13 rotiert gemäß Figur 2 im Uhrzeigersinn und somit entgegen ihrer Förderrichtung, in der sie während der Blutbehandlung fördert, und die Substituatpumpe 23 rotiert entgegen dem Uhrzeigersinn und somit in ihrer Förderrichtung, in der sie auch während der Blutbehandlung fördert. Über die Leitung 27 wir Substitutionslösung eingebracht. Dazu wird das Prädilutionszugabeventil 291 der Prädilutionszugabestelle 29. Alternativ oder zusätzlich ist auch eine Zugabe an einer Postdilutionszugabestelle 31 denkbar. In der hier gezeigten Ausführungsform ist das Postdilutionszugabeventil 311 geschlossen.

Aufgrund des Betriebes der Blutpumpe 13 entgegen ihrer Förderrichtung, die sie während der Blutbehandlung aufweist, fördert die Blutpumpe 13 bei der Reinfusion das Blut in den Patienten zurück, das sich in dem Abschnitt 1 des extrakorporalen Kreislaufes zwischen der Blutpumpe und dem Zugang 5 befindet. Die Substituatpumpe 23 fördert Substitutionsflüssigkeit durch den verbleibenden Teil des extrakorporalen Blutkreislaufes einschließlich des Dialysators 15 und verdrängt dabei das in diesem Teil befindliche Blut. Dieses gelangt über den venösen Zugang zurück zum Patienten.

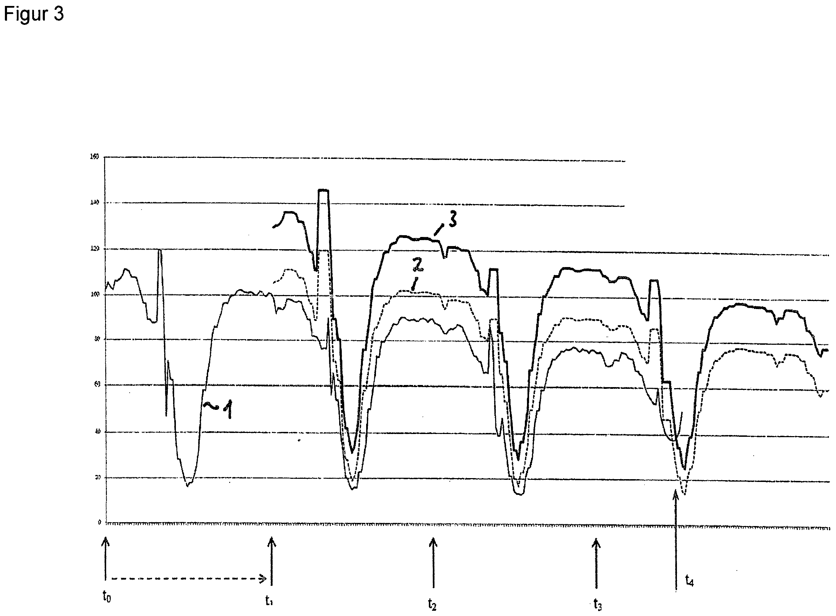

Figur 3 zeigt einen Druckverlauf, bei dem ein Druckanstieg, der zum Zeitpunkt t₄ durch den Sensor P gemessen wird und der durch ein Gerinnsel verursacht ist.

Die Zeiträume t₀ - t₁, t₁ - t₂, t₂ - t₃ t₃ etc. beschreiben jeweils den Aktivitätszeitraum eines fördernden Elementes, d.h. jeweils einen Förderzyklus, d.h. eine Förderperiode der Pumpe B. Wie dies aus Figur 3 hervorgeht ist der Druck in seinem Verlauf insgesamt abnehmend, da das Gemisch aus Blut und Austauschflüssigkeit im Laufe der Reinfusion einen geringer werdenden Gehalt an Blut aufweist, wobei die Viskosität der Flüssigkeit abnimmt und bei gleichbleibendem Förderverhalten der Pumpe sich im Systemschlauch ein abnehmender Druck einstellt.

Das Verfahren beginnt zum Start der Reinfusion, d.h. zum Zeitpunkt t₀. Die Pumpe B fördert von der Behandlungsvorrichtung D zum Patientenanschluss A1. Dabei entsteht in der arteriellen Leitung A ein typischer Druck, der fortwährend in der Auswerteeinheit E aufgezeichnet wird.

Zum Zeitpunkt t₁ ist ein vollständiger Förderzyklus bzw. Aktivitätszyklus eines einzelnen fördernden Elementes aufgezeichnet worden.

Den Abschluss eines Förderzyklus erfasst die Auswerteeinheit E anhand der Daten des Drehwinkelsensors α der Pumpe B, der mechanisch mit der Pumpe B verbunden ist bzw. einen Bestandteil der Pumpe B bildet. Alternativ ist eine Erfassung des Abschlusses eines Förderzyklus durch Erfassung des Druckverlaufes oder durch Berechnung mittels Pumpengeschwindigkeit und der Zeit oder dem Drehwinkel des Rotors und der Übersetzung des Getriebes denkbar und von der Erfindung mit umfasst.

Für den nachfolgenden Förderzyklus in der Zeitspanne t₁ - t₂ wird der in dem ersten Förderzyklus aufgenommen Druckwert ggf. zzgl. eines Zuschlages als Alarmschwelle definiert.

In Figur 1 ist der gemessene arterielle Druck mit dem Bezugszeichen 1, der arterielle Druck in dem jeweils vorausgegangenen Rollendurchgang bzw. Förderzyklus mit dem Bezugszeichen 2 und der Schwellenwert bzw. die Alarmschwelle mit dem Bezugszeichen 3 gekennzeichnet. Das Bezugszeichen 3 kennzeichnet somit den Referenzdruckverlauf, mit dem der aktuelle Druckverlauf 1 verglichen wird.

Für das Intervall t₁ - t₂ liegt somit nunmehr eine Alarmschwelle 3 vor, die jeweils mit dem aktuellen Druck 1 in diesem Zeitintervall verglichen wird. Der jeweils aktuelle Druck 1, der sich typischerweise im Bereich von 0 - 150 mmHg bewegt, wird mit der Alarmschwelle 3, die üblicherweise im Bereich von 0 - 225 mmHg liegt, verglichen.

Übersteigt der aktuelle Druck 1 die Alarmschwelle, d.h. den Referenzdruckverlauf 3, wird die Förderung der Flüssigkeit in der arteriellen Leitung A durch Schließen der Klemme F sofort gestoppt.

Die Alarmschwelle 3 wird von der Auswerteeinheit E so berechnet, dass der Druck zur selben Zeit innerhalb des Aktivitätszyklus des vorausgegangenen Förderelementes bzw. Förderzyklus herangezogen wird und ggf. zusätzlich um einen Faktor zum höheren Druck hin verschoben wird (Offset). Dieser Faktor kann im Bereich von 5% bis 50% liegen. Dieser ermöglicht, dass Unterschiede zwischen den zwei fördernden Elementen keine Fehlalarme erzeugen.

Grundsätzlich ist auch ein negativer Offset möglich und von der Erfindung umfasst, um den Schwellenwert bei starkem Abfall des Druckes zwischen den einzelnen Zyklen entsprechend anzupassen.

Bezogen auf das Ausführungsbeispiel in Figur 3 wird somit die Alarmschwelle 3 für das Intervall t₁ - t₂ aus dem Druckverlauf des vorausgegangenen Intervalls t₀ - t₁ zuzüglich eines Aufschlages berechnet.

Wie dies weiter aus Figur 3 hervorgeht, beginnen die Kurven 2 und 3 für das Zeitintervall t₀ - t₁ zeitversetzt. Um auch für diesen Startbereich t₀ - t₁ einen Referenzwert definieren zu können, wird in einer Ausgestaltung der Erfindung der Referenzwert auf einen festen Wert bzw. Wertverlauf oder auf einen Erfahrungswert von früheren Messungen gesetzt.

Das Ausführungsbeispiel bezieht sich auf eine Schlauchrollenpumpe mit zwei fördernden Elementen, d.h. mit zwei Rollen, die relativ zur Drehachse gegenüberliegend angeordnet sind. Grundsätzlich ist die Erfindung nicht darauf nicht beschränkt, sondern umfasst jede zyklisch arbeitende Pumpe, wie bspw. auch eine peristaltische Pumpe mit mehr als zwei fördernden Elementen.

Die Druckmessung kann in jeglicher Patientenleitung des extrakorporalen Kreislaufes vorgenommen werden, bspw. auch nach dem Gerinnselfilter in der venösen Leitung, um dort gewachsene Gerinnsel zu erkennen.

Besonders vorteilhaft ist jedoch die in Figur 1 dargestellte Anordnung mit der Druckerfassung und Klemme in der arteriellen Leitung.

Wie oben ausgeführt, wird auf die Druckkurve vom vorausgegangenen Rollendurchgang bzw. -zyklus noch einige mmHg aufaddiert, um die tatsächliche Alarmschwelle, d.h. den Referenzdruckverlauf festzulegen.

Liegt der gemessene Druckwert 1 auf oder über dem Referenzdruckwert 3 kommen als mögliche Maßnahmen z. B. das Schließen der Klemme F, das Stoppen der Pumpe B, die Fortführung der Reinfusion mittels eines Verfahrens, bei dem ein Behälter mit Austauschflüssigkeit an Anschluss A1 angeschlossen wird und diese anschließend von der Pumpe B (vorwärts) in Richtung der Behandlungseinrichtung gefördert wird, wie etwa die Fortführung der Reinfusion von nur noch NaCl und das Verhindern der Rückwärtsförderung der Pumpe B oder eine Kombination dieser Maßnahmen in Betracht.

## Patentansprüche

1. Vorrichtung zur druckbasierten Erkennung eines Gerinnsels in einem extrakorporalen Blutkreislauf, umfassend:
eine zyklisch arbeitende Pumpe (B) zur Förderung der in dem extrakorporalen Kreislauf befindlichen Flüssigkeit,
einen Druckaufnehmer (P), der angeordnet ist, um in einem aktuellen Förderzyklus der Pumpe (B) den aktuellen Druckverlauf (1) über die Zeit in der von der Pumpe (B) geförderten Flüssigkeit zu ermitteln,
einen Speicher, in dem wenigstens ein Referenzdruckverlauf (3) abgespeichert ist, der auf einem Druckverlauf (2) über die Zeit basiert, der in einem dem aktuellen Förderzyklus der Pumpe (B) vorausgehenden Förderzyklus der Pumpe (B) ermittelt wurde, und
eine Auswerteeinheit (E), die derart ausgebildet ist, dass diese entsprechende Zeitpunkte des Referenzdruckverlaufs (3) mit entsprechenden Zeitpunkten des aktuellen Druckverlaufes (1) vergleicht,
wobei die Vorrichtung basierend auf diesem Vergleich ein Gerinnsel erkennt.

2. Vorrichtung nach Anspruch 1, **dadurch gekennzeichnet, dass** die Auswerteeinheit (E) oder eine sonstige Einheit der Vorrichtung derart ausgebildet ist, dass diese wenigstens eine Maßnahme vornimmt, wenn der aktuelle Druckverlauf (1) den Referenzdruckverlauf (3) erreicht oder diesen übersteigt.

3. Vorrichtung nach Anspruch 1 oder 2, **dadurch gekennzeichnet, dass** es sich bei der Pumpe (B) um eine Pumpe handelt, die eine periodisch schwankende Förderrate aufweist, so dass sich in der von der Pumpe geförderten Flüssigkeit ein zyklischer Druckverlauf ergibt, wobei vorzugsweise vorgesehen ist, dass es sich bei der Pumpe um eine peristaltische Pumpe oder um eine Membranpumpe handelt.

4. Vorrichtung nach einem der vorhergehenden Ansprüche, **dadurch gekennzeichnet, dass** der Referenzdruckverlauf (3) auf einem gegenüber dem aktuellen Druckverlauf (1) phasenverschobenen Druckverlauf (2) basiert und/oder dass der Referenzdruckverlauf (3) auf dem dem aktuellen Druckverlauf (1) unmittelbar vorausgegangenen und/oder auf einem weiter zurückliegenden Druckverlauf (2) basiert.

5. Vorrichtung nach einem der vorhergehenden Ansprüche, **dadurch gekennzeichnet, dass** der Referenzdruckverlauf (3) durch einen in einem vorausgegangen Förderzyklus der Pumpe (B) ermittelten Druckverlauf (2) zuzüglich eines Zuschlages auf diesen Druckverlauf (2) gebildet wird.

6. Vorrichtung nach einem der vorhergehenden Ansprüche, **dadurch gekennzeichnet, dass** der extrakorporale Kreislauf wenigstens eine arterielle Leitung (A) und wenigstens eine venöse Leitung (V) aufweist und dass der Druckaufnehmer (P) in der arteriellen (A) und/oder in der venösen Leitung (V) angeordnet ist, und wobei vorzugsweise -in der arteriellen (A) oder venösen Leitung (V), in der sich der Druckaufnehmer (P) befindet, kein Gerinnselfänger (C) angeordnet ist oder dass sich in der arteriellen (A) und/oder in der venösen Leitung (V) ein Gerinnselfänger befindet.

7. Vorrichtung nach einem der Ansprüche 2 bis 6, **dadurch gekennzeichnet, dass** die Auswerteeinheit (E) oder die sonstige Einheit derart ausgebildet ist, dass diese als Maßnahme wenigstens ein im extrakorporalen Kreislauf befindliches Ventil (F) schließt und/oder die Pumpe (B) stoppt und/oder einen Hinweis an den Nutzer des Systems ausgibt.

8. Vorrichtung nach einem der vorhergehenden Ansprüche, **dadurch gekennzeichnet, dass** die Vorrichtung wenigstens einen Sensor zur Erfassung der Position des oder der Förderorgane der Pumpe (B) aufweist, wobei der Sensor zum zeitentsprechenden Vergleich zwischen dem aktuellen Druckverlauf (1) und dem Referenzdruckverlauf (3) mit der Auswerteeinheit (E) verbunden ist.

9. System umfassend wenigstens ein Blutbehandlungsgerät, das den extrakorporalen Kreislauf aufweist, sowie umfassend wenigstens eine Vorrichtung zur druckbasierten Erkennung eines Gerinnsels gemäß einem der Ansprüche 1 bis 8, wobei vorzugsweise-der extrakorporale Kreislauf mit wenigstens einem Konnektor (A1, A2) zur Verbindung des extrakorporalen Kreislaufes mit einem Patienten versehen ist.

10. System nach Anspruch 9, **dadurch gekennzeichnet, dass** es sich bei dem Blutbehandlungsgerät um ein Hämofiltrations-, Hämodialyse-, Hämodiafiltrations-, Apheresegerät oder um eine Vorrichtung zur Medikation handelt.

## Claims

1. Apparatus for a pressure-based detection of a clot in an extracorporeal blood circuit, comprising:
a cyclically operating pump (B) for conveying the fluid located in the extracorporeal circuit,
a pressure sensor (P) which is arranged to determine the current pressure development (1) over time in the fluid conveyed by the pump (B) in a current conveying cycle of the pump (B),
a memory in which at least one reference pressure development (3) is stored which is based on at least one pressure development (2) over time which was determined in a conveying cycle of the pump (B) preceding the current conveying cycle of the pump (B), and
an evaluation unit (E) which is configured such that it compares the current pressure development (1) with the reference pressure development (3) over a corresponding time,
wherein the apparatus detects a clot based on this detection.

2. Apparatus in accordance with claim 1, **characterized in that** the evaluation unit (E) or another unit of the apparatus is configured such that it carries out at least one measure when the current pressure development (1) reaches or exceeds the reference pressure development (3).

3. Apparatus in accordance with claim 1 or claim 2, **characterized in that** the pump (B) is pump which has a periodically fluctuating conveying rate so that a cyclic pressure development results in the fluid conveyed by the pump, with provision preferably being made that the pump is a peristaltic pump or a membrane pump.

4. Apparatus in accordance with one of the preceding claims, **characterized in that** the reference pressure development (3) is based on a pressure development (2) phase-shifted with respect to the current pressure development (1); and/or **in that** the reference pressure development (3) is based on the pressure development (2) directly preceding the current pressure development (1) and/or on a pressure development (2) further in the past.

5. Apparatus in accordance with one of the preceding claims, **characterized in that** the reference pressure development (3) is formed by a pressure development (2) determined in a preceding conveying cycle of the pump (B), plus a supplement on this pressure development (2).

6. Apparatus in accordance with one of the preceding claims, **characterized in that** the extracorporeal circuit has at least one arterial line (A) and at least one venous line (V); and **in that** the pressure sensor (P) is arranged in the arterial line (A) and/or in the venous line (V) and wherein preferably no clot catcher (C) is arranged in the arterial line (A) or in the venous line (V) in which the pressure sensor (P) is arranged; or **in that** a clot catcher is located in the arterial line (A) and/or in the venous line (V).

7. Apparatus in accordance with one of the claims 2 to 6, **characterized in that** the evaluation unit (E) or the other unit is configured such that as a measure it closes at least one valve (F) located in the extracorporeal circuit and/or stops the pump (B) and/or outputs a signal to the user of the system.

8. Apparatus in accordance with one of the preceding claims, **characterized in that** the apparatus has at least one sensor for detecting the position of the conveying member or members of the pump (B), with the sensor being connected to the evaluation unit (E) for a comparison corresponding in time between the current pressure development (1) and the reference pressure development (3).

9. System comprising at least one blood treatment unit which has the extracorporeal circuit and comprising at least one apparatus for a pressure-based detection of a clot in accordance with one of the claims 1 to 8, wherein preferably the extracorporeal circuit is provided with at least one connector (A1, A2) for connecting the extracorporeal circuit to a patient.

10. System in accordance with claim 9, **characterized in that** the blood treatment unit is a hemofiltration unit, a hemodialysis unit, a hemodiafiltration unit, an apheresis unit or an apparatus for medicating.

## Revendications

1. Dispositif pour la détection basée sur la pression d'un caillot dans un circuit sanguin extracorporel, comprenant :
une pompe (B) fonctionnant de manière cyclique, destinée au transport du liquide se trouvant dans le circuit extracorporel,
un capteur de pression (P), qui est disposé de façon à déterminer, dans un cycle de transport actuel de la pompe (B), la courbe de pression (1) actuelle en fonction du temps dans le liquide transporté par la pompe (B),
une mémoire, dans laquelle au moins une courbe de pression de référence (3) est enregistrée, laquelle est basée sur une courbe de pression (2) en fonction du temps qui a été déterminée dans un cycle de transport de la pompe (B) précédant le cycle de transport actuel de la pompe (B), et
une unité d'évaluation (E), qui est conçue de manière à comparer des instants correspondants de la courbe de pression de référence (3) à des instants correspondants de la courbe de pression (1) actuelle,
le dispositif détectant un caillot sur la base de cette comparaison.

2. Dispositif selon la revendication 1, **caractérisé en ce que** l'unité d'évaluation (E) ou une autre unité du dispositif est conçue de manière à prendre au moins une mesure quand la courbe de pression (1) actuelle atteint ou dépasse la courbe de pression de référence (3).

3. Dispositif selon la revendication 1 ou 2, **caractérisé en ce que** la pompe (B) est une pompe qui présente un débit variable de manière périodique, de telle sorte qu'il en résulte une courbe de pression cyclique dans le liquide transporté par la pompe, la pompe étant de préférence prévue sous la forme d'une pompe péristaltique ou d'une pompe à membrane.

4. Dispositif selon l'une des revendications précédentes, **caractérisé en ce que** la courbe de pression de référence (3) se base sur une courbe de pression (2) déphasée par rapport à la courbe de pression (1) actuelle et/ou **en ce que** la courbe de pression de référence (3) se base sur une courbe de pression (2) précédant directement la courbe de pression (1) actuelle et/ou sur une courbe de pression (2) plus ancienne.

5. Dispositif selon l'une des revendications précédentes, **caractérisé en ce que** la courbe de pression de référence (3) est formée par une courbe de pression (2) déterminée dans un cycle de transport précédent de la pompe (B) avec en plus un supplément à cette courbe de pression (2).

6. Dispositif selon l'une des revendications précédentes, **caractérisé en ce que** le circuit extracorporel présente au moins une ligne artérielle (A) et au moins une ligne veineuse (V) et **en ce que** le capteur de pression (P) est disposé dans la ligne artérielle (A) et/ou dans la ligne veineuse (V), et dans lequel aucun piège à caillots (C) n'est de préférence disposé dans la ligne artérielle (A) ou veineuse (V) dans laquelle se trouve le capteur de pression (P), ou **en ce qu'**un piège à caillots se trouve dans la ligne artérielle (A) et/ou dans la ligne veineuse (V).

7. Dispositif selon l'une des revendications 2 à 6, **caractérisé en ce que** l'unité d'évaluation (E) ou l'autre unité est conçue de manière à, en guise de mesure, au moins fermer une soupape (F) se trouvant dans le circuit extracorporel et/ou arrêter la pompe (B) et/ou fournir une indication à l'utilisateur du système.

8. Dispositif selon l'une des revendications précédentes, **caractérisé en ce que** le dispositif présente au moins un capteur destiné à la détection de la position du ou des organes de transport de la pompe (B), le capteur étant relié à l'unité d'évaluation (E) pour la comparaison à instants correspondants entre la courbe de pression (1) actuelle et la courbe de pression de référence (3).

9. Système comprenant au moins un appareil de traitement du sang qui présente le circuit extracorporel, et comprenant au moins un dispositif pour la détection basée sur la pression d'un caillot selon l'une des revendications 1 à 8, le circuit extracorporel étant de préférence pourvu d'au moins un connecteur (A1, A2) destiné au raccordement du circuit extracorporel à un patient.

10. Système selon la revendication 9, **caractérisé en ce que** l'appareil de traitement du sang est un appareil d'hémofiltration, d'hémodialyse, d'hémodiafiltration, d'aphérèse ou un dispositif de médication.
